# EUROPEAN PATENT APPLICATION

(11) **EP 4 534 643 A1**
(43) Date of publication of application: **09.04.2025**
(21) Application number: 24204836.1
(22) Date of filing: 04.10.2024
(51) Int. Cl.: C12M 3/00, C12M 1/00, C12M 1/34

(54) **CULTURING SYSTEM**

(30) Priority: 06.10.2023 JP 2023174554; 02.10.2024 JP 2024173870
(71) Applicant: CANON KABUSHIKI KAISHA, Tokyo 146-8501 (JP); Canon Medical Systems Corporation, Tochigi 324-0036 (JP)
(72) Inventor: YAMAKI, Tomoaki, Tokyo, 146-8501 (JP); UKIYO, Noritaka, Tokyo, 146-8501 (JP); YANAGITA, Kazutaka, Tokyo, 146-8501 (JP); YAMAGUCHI, Yosuke, Otawara-shi, 324-0036 (JP); TAHARA, Hirotoshi, Otawara-shi, 324-0036 (JP)
(74) Representative: Marks & Clerk LLP

(57) **Abstract**

A culturing system (1, 1a, 1b) according to an embodiment includes an incubator (30) and a cartridge (20).

The incubator (30) has an internal space (30e) and is capable of adjusting one or both of a temperature and a humidity of gas in the internal space (30e). The cartridge (20) is capable of accommodating a culturing container (20f) used for culturing a cell, a culture medium keeping unit (20b) configured to keep therein a culture medium kept cold, and a flow path (20d) which connects the culturing container (20f) to the culture medium keeping unit (20b) and through which the culture medium kept in the culture medium keeping unit (20b) flows toward the culturing container (20f). In the culturing system (1, 1a, 1b), the gas in the internal space (30e) is supplied to the culturing container (20f), by inserting a part of the cartridge (20) into the internal space (30e).

## Description

### FIELD

Embodiments described herein relate generally to a culturing system.

### BACKGROUND

There is a demand for culturing systems capable of culturing cells with high quality. However, to culture the cells, it is necessary to keep the temperature of a culturing container used for culturing the cells at a temperature (e.g., 37 degrees centigrade) suitable for the cell culturing. In addition, it is necessary to reserve a replacement-purpose culture medium which needs to be used as a replacement during the culturing at a temperature (e.g., 5 degrees centigrade) different from the abovementioned temperature suitable for the cell culturing. Accordingly, the replacement-purpose culture medium needs to be held in a different environment from that of the culturing container. Consequently, at the time of replacing a culture medium in the culturing container, there is a risk of contamination. If contamination occurs, it will be difficult to culture the cells with high quality.

### SUMMARY OF THE INVENTION

A culturing system provided in at least one aspect of the present disclosure includes an incubator and a cartridge. The incubator has an internal space and is capable of adjusting one or both of a temperature and a humidity of gas in the internal space. The cartridge is capable of accommodating a culturing container used for culturing a cell, a culture medium keeping unit configured to keep therein a culture medium kept cold, and a flow path which connects the culturing container to the culture medium keeping unit and through which the culture medium kept in the culture medium keeping unit flows toward the culturing container. In the culturing system, the gas in the internal space is supplied to the culturing container, by inserting a part of the cartridge into the internal space.

The cartridge may include a thermal insulation unit. The thermal insulation unit may be provided between the culturing container and the culture medium keeping unit and configured to isolate the culturing container from the culture medium keeping unit.

The thermal insulation unit may be formed while including a partition wall and a thermal insulation member configured to isolate the culturing container from the culture medium keeping unit.

The incubator may include a seal structure provided at an opening part being a boundary part between the internal space and an outside of the incubator.

The incubator may include one or both of a thermal insulation structure and a heating structure provided at an opening part being a boundary part between the internal space and an outside of the incubator.

The incubator may include a shutter configured, while the cartridge is not inserted in the internal space, to close an opening of an opening part being a boundary part between the internal space and an outside of the incubator.

Inserted into the incubator may be a member configured, while the cartridge is not inserted in the internal space, to close an opening of an opening part being a boundary part between the internal space and an outside of the incubator.

The incubator may include a regulating member configured, while the cartridge is inserted in the internal space, to regulate a movement in an insertion direction of the cartridge, so that a thermal insulation unit is positioned more outward than the seal structure, the thermal insulation unit being included in the cartridge, being provided between the culturing container and the culture medium keeping unit, and being configured isolate the culturing container from the culture medium keeping unit.

The cartridge may include a gas supply port used for bringing the gas in the internal space into the culturing container.

The cartridge may include a gas discharge port used for discharging the gas from the inside of the culturing container.

The flow path may be provided with a storage unit configured to warm the culture medium kept cold in the culture medium keeping unit.

The cartridge may include a cold storage unit configured to keep cold the culture medium kept in the culture medium keeping unit.

A cold storage apparatus may further be provided, and the cold storage apparatus may be configured, in the state where the cartridge is inserted in the cold storage apparatus, to keep cold the culture medium kept in the culture medium keeping unit.

An observation apparatus may further be provided so as to be used at a time of observing an inside of the culturing container, through an observation window included in the incubator.

The incubator may include a first thermometer configured to measure a first temperature of the internal space; a hygrometer configured to measure the humidity of the internal space; a second thermometer configured to measure a second temperature of the seal structure; a heater configured to heat the seal structure; a calculating unit configured to calculate a dew point of the internal space on the basis of the first temperature measured by the first thermometer and the humidity measured by the hygrometer; and a controlling unit configured to control the heater so that the second temperature measured by the second thermometer becomes equal to or higher than the dew point.

The controlling unit may be configured to control the heater so that the second temperature measured by the second thermometer becomes equal to or higher than the dew point and equal to or lower than the first temperature measured by the first thermometer.

The first thermometer may be provided in a position within the internal space while being positioned apart from the seal structure by a distance in a range from 35 millimeters or more to 100 millimeters or less inclusive, and the hygrometer may be provided in a position within the internal space while being positioned apart from the seal structure by a distance in a range from 35 millimeters or more to 100 millimeters or less inclusive.

The incubator may include: a gas introduction port through which the gas of which the one or both of the temperature and the humidity has been adjusted is introduced; and a fan including a suction port through which the gas is sucked from an inside of the internal space and a blow-out port through which the sucked gas is blown out. The first thermometer may be provided in a position within the internal space, while being positioned apart from the gas introduction port by a distance in a range from 30 millimeters or more to 80 millimeters or less inclusive or being positioned apart from the blow-out port by a distance in a range from 30 millimeters or more to 80 millimeters or less inclusive. The hygrometer may be provided in a position within the internal space, while being positioned apart from the gas introduction port by a distance in a range from 30 millimeters or more to 80 millimeters or less inclusive or being positioned apart from the blow-out port by a distance in a range from 30 millimeters or more to 80 millimeters or less inclusive.

The first thermometer may be provided in a position closer to the seal structure than the hygrometer is.

In the state where the cartridge is inserted in the internal space, the second thermometer may be positioned apart from an abutment part where the cartridge abuts against the seal structure, by a distance in a range from 10 millimeters or more to 20 millimeters or less inclusive.

The seal structure may include a plurality of seal structures respectively corresponding to a plurality of spaces included in the internal space. The incubator may include: a plurality of first thermometers each of which is configured to measure the first temperature of a corresponding one of the plurality of spaces; a plurality of hygrometers each of which is configured to measure the humidity of a corresponding one of the plurality of spaces; a plurality of second thermometers each of which is configured to measure the second temperature of a corresponding one of the plurality of seal structures; a plurality of heaters each of which is configured to heat a corresponding one of the plurality of seal structures; a calculating unit configured to calculate a dew point of each of the plurality of spaces, on the basis of the measured first temperature and the measured humidity; and a controlling unit configured to control each of the plurality of heaters so that the second temperature measured with respect to each of the plurality of seal structures becomes equal to or higher than a corresponding one of the dew points.

### BRIEF DESCRIPTION OF THE DRAWINGS

FIG. 1 is a drawing illustrating an exemplary configuration of a culturing system according to a first embodiment;
FIG. 2 is a diagram illustrating an example of an internal structure of a cartridge according to the first embodiment;
FIG. 3 is a diagram illustrating an example of an internal structure of an incubator according to the first embodiment;
FIG. 4 is a diagram illustrating an example of the incubator according to the first embodiment, as viewed from the side of a slot (hereinafter, "slot side");
FIG. 5 is a cross-sectional view of the incubator according to the first embodiment taken on a plane going through an observation window, illustrating an example of the incubator as viewed from the side opposite from the slot side;
FIG. 6 is a diagram illustrating an example of an internal structure of the culturing system in a state where a part of the cartridge is inserted in the incubator according to the first embodiment;
FIG. 7 is a diagram illustrating an exemplary configuration of a seal structure according to the first embodiment;
FIG. 8 is a cross-sectional view of an example of the culturing system taken on a plane going through the observation window, in a state where a part of the cartridge is inserted in the incubator according to the first embodiment, illustrating an example of the incubator as viewed from the side opposite from the slot side;
FIG. 9 is a diagram illustrating an example of an internal structure of an incubator in a culturing system according to a first modification example;
FIG. 10 is a diagram illustrating an example of the incubator according to the first modification example, as viewed from the slot side;
FIG. 11 is a diagram illustrating an example of an internal structure of an incubator in a culturing system according to a second modification example;
FIG. 12 is a diagram illustrating an example of an internal structure of an incubator in a culturing system according to a third modification example;
FIG. 13 is a diagram illustrating an example of an internal structure of a cartridge according to a fourth modification example;
FIG. 14 is a diagram illustrating an example of an internal structure of a cartridge according to a fifth modification example;
FIG. 15 is a diagram illustrating an exemplary configuration of a culturing system according to a second embodiment;
FIG. 16 is a flowchart illustrating a flow in a temperature controlling process executed by the culturing system according to the second embodiment;
FIG. 17 is a diagram illustrating an exemplary configuration of a culturing system according to a modification example of the second embodiment; and
FIG. 18 is a diagram illustrating an example of the incubator according to the modification example of the second embodiment, as viewed from the slot side.

### DETAILED DESCRIPTION

One of the problems to be solved by the embodiments set forth in the present disclosure is to culture cells with high quality. However, problems that can possibly be solved by the embodiments of the present disclosure are not limited to the abovementioned problem. It is also possible to consider problems corresponding to advantageous effects of the configurations described in the following embodiments as other problems.

A culturing system according to an embodiment includes an incubator and a cartridge. The incubator has an internal space and is capable of adjusting one or both of a temperature and a humidity of gas in the internal space.

The cartridge is capable of accommodating a culturing container used for culturing a cell, a culture medium keeping unit configured to keep therein a culture medium kept cold, and a flow path which connects the culturing container to the culture medium keeping unit and through which the culture medium kept in the culture medium keeping unit flows toward the culturing container. In the culturing system, the gas in the internal space is supplied to the culturing container, by inserting a part of the cartridge into the internal space.

Exemplary embodiments of the present disclosure will be explained in detail below, with reference to the accompanying drawings. The following embodiments are not intended to limit the present disclosure set forth in the claims. Also, not all the combinations of the features described in the present embodiments are necessarily requisite for the problem solving means of the present disclosure.

### First Embodiment

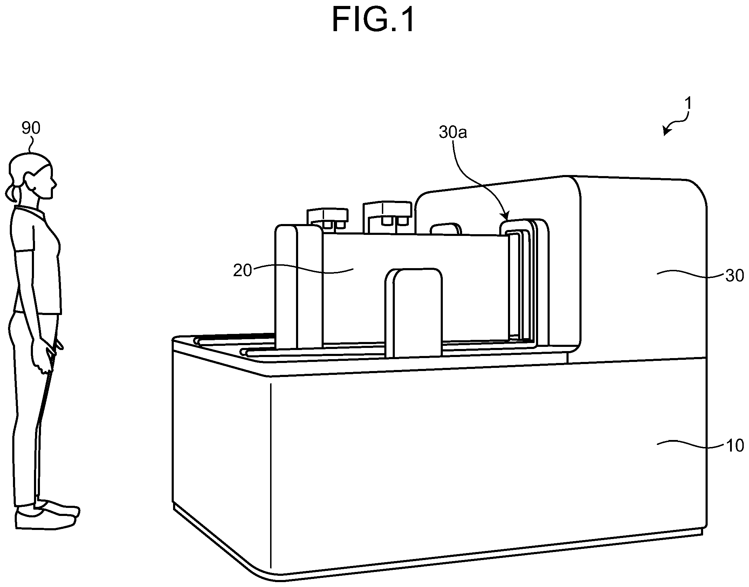

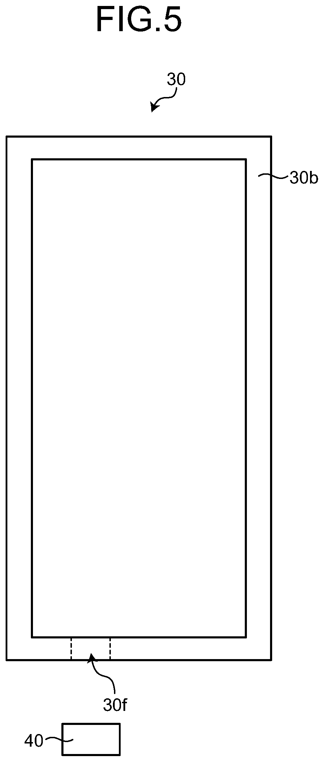

A culturing system according to a first embodiment will be explained. FIG. 1 is a drawing illustrating an exemplary configuration of a culturing system 1 according to the first embodiment. As illustrated in FIG. 1, the culturing system 1 includes a base 10, a cartridge 20, and an incubator 30. Further, the culturing system 1 also includes an observation apparatus 40 (see FIG. 5). In the culturing system 1, as a result of the incubator 30 supplying gas suitable for the cell culturing to a culturing container provided in the cartridge 20, the cells in the culturing container are cultured.

The base 10 is a base configured to support the incubator 30 and to have the cartridge 20 placed thereon.

The cartridge 20 placed on the base 10 is capable of moving over the base 10. The cartridge 20 may be inserted into a slot (an insertion opening) 30a of the incubator 30 manually by a user 90 or may be inserted into the slot 30a automatically by a moving mechanism (not illustrated).

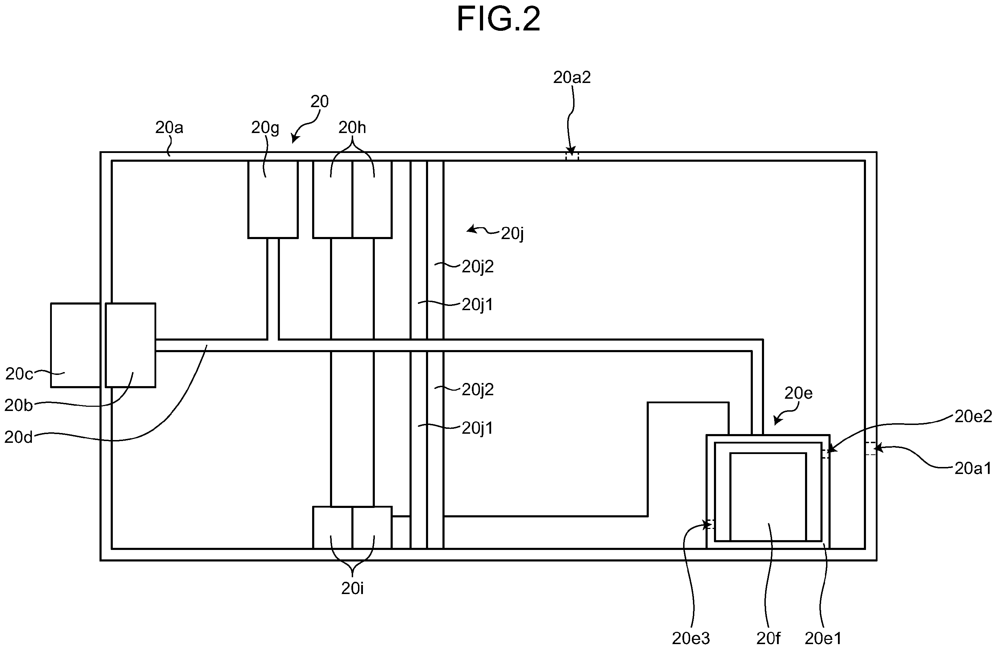

The cartridge 20 is configured to house therein the culturing container containing the cells to be cultured. FIG. 2 is a diagram illustrating an example of an internal structure of the cartridge 20 according to the first embodiment. As illustrated in FIG. 2, the cartridge 20 includes a casing 20a, a culture medium keeping unit 20b, a cold storage unit 20c, a flow path 20d, a culturing unit 20e, a culturing container 20f, a liquid delivery unit 20g, a chemical liquid mount unit 20h, a pre-processing unit 20i, and a thermal insulation unit 20j. In the present example, FIG. 2 is a front view of the cartridge 20, as seen through a front face of the casing 20a of the cartridge 20.

The cartridge 20 has a substantially cuboidal shape. As illustrated in FIG. 2, in a frontal view, the casing 20a of the cartridge 20 has a substantially rectangular shape. Within an internal space of the cartridge 20 formed by the casing 20a, the cartridge 20 houses the culture medium keeping unit 20b, the flow path 20d, the culturing unit 20e, the culturing container 20f, the liquid delivery unit 20g, the chemical liquid mount unit 20h, the pre-processing unit 20i, and the thermal insulation unit 20j. In addition, the cold storage unit 20c is provided on the outside of the casing 20a.

In this situation, the abovementioned plurality of elements structuring the cartridge 20 may be integrally formed. Alternatively, the plurality of elements may be manufactured separately, so that the cartridge 20 houses therein the plurality of elements.

The casing 20a has formed therein a gas supply port 20a1 used for bringing gas in an internal space of the incubator 30 into the culturing container 20f. In addition, the casing 20a has formed therein a gas discharge port 20a2 used for discharging gas from the inside of the culturing container 20f.

The culture medium keeping unit 20b is configured to keep a culture medium to replace another culture medium provided in the culturing container 20f. In other words, the culture medium keeping unit 20b has stored therein the replacement-purpose culture medium. For example, the culture media used in the present embodiment are liquid culture media.

The cold storage unit 20c is provided in a position where it is possible to keep cool the culture medium kept in the culture medium keeping unit 20b and is configured to keep the culture medium cool so that the culture medium kept in the culture medium keeping unit 20b is kept at a temperature (e.g., 5 degrees centigrade) suitable for keeping the culture medium. In this manner, the cold storage unit 20c is able to have the culture medium kept in the culture medium keeping unit 20b at the temperature suitable for keeping the culture medium. For example, the cold storage unit 20c is provided in a position adjacent to the culture medium keeping unit 20b, via the casing 20a. For example, the cold storage unit 20c may be realized by using a Peltier module or the like.

The flow path 20d is connected to the culture medium keeping unit 20b, the culturing unit 20e, and the liquid delivery unit 20g and is a closed-system flow path in which the culture medium kept in the culture medium keeping unit 20b flows toward the culturing container 20f provided in the culturing unit 20e. In the present example, the closed-system flow path denotes, for instance, a flow path configured so that the culture medium flowing through the flow path is prevented from leaking to the outside of the cartridge 20.

The liquid delivery unit 20g is configured to deliver the culture medium kept in the culture medium keeping unit 20b to the culturing container 20f provided in the culturing unit 20e, via the flow path 20d. In the present embodiment, the liquid delivery unit 20g is configured to suck the culture medium from the culture medium keeping unit 20b via the flow path 20d. After that, the liquid delivery unit 20g is configured to deliver the sucked culture medium to the culturing container 20f provided in the culturing unit 20e via the flow path 20d. For example, the liquid delivery unit 20g includes a culture medium storage container for storing the culture medium therein and a pressure controlling mechanism configured to control pressure in the culture medium storage container. By causing the pressure controlling mechanism to decrease the pressure in the culture medium storage container, the liquid delivery unit 20g is configured to suck the culture medium from the culture medium keeping unit 20b via the flow path 20d and to further put the culture medium into the culture medium storage container. Also, by causing the pressure controlling mechanism to increase the pressure in the culture medium storage container, the liquid delivery unit 20g is configured to deliver the culture medium in the culture medium storage container to the culturing container 20f provided in the culturing unit 20e via the flow path 20d. In addition, before the culture medium is delivered to the culturing container 20f, an older culture medium in the culturing container 20f may be moved to a collection container (not illustrated).

The chemical liquid mount unit 20h has various types of chemical liquids mounted thereon and is configured to supply any of the various types of chemical liquids to the pre-processing unit 20i. By using the supplied chemical liquid, the pre-processing unit 20i is configured to perform various types of pre-processing processes such as selecting cells suitable for the culturing process and to further deliver the pre-processed cells to the culturing container 20f provided in the culturing unit 20e. For example, the pre-processing unit 20i is configured to deliver the selected cells to the culturing container 20f.

The culturing unit 20e is configured to house the culturing container 20f in a space formed by a partition wall 20e1. The partition wall 20e1 has formed therein a gas supply port 20e2 used for bringing gas from the internal space of the cartridge 20 into the culturing container 20f. In addition, the partition wall 20e1 has formed therein a gas discharge port 20e3 used for discharging gas from the inside of the culturing container 20f.

The culturing container 20f is configured to culture the cells in the culturing container 20f, by using the culture medium in the culturing container 20f.

The thermal insulation unit 20j is provided so as to isolate the space in which the culturing container 20f is present, from the space in which the culture medium keeping unit 20b is present and is configured to inhibit thermal conduction between the space in which the culturing container 20f is present and the space in which the culture medium keeping unit 20b is present. In other words, the thermal insulation unit 20j is provided between the culturing container 20f and the culture medium keeping unit 20b and is configured to prevent heat from moving between the space in which the culturing container 20f is present and the space in which the culture medium keeping unit 20b is present, by isolating the culturing container 20f from the culture medium keeping unit 20b.

The thermal insulation unit 20j is formed while including a partition wall 20j1 isolating the culturing container 20f from the culture medium keeping unit 20b; and a thermal insulation member 20j2 isolating the culturing container 20f from the culture medium keeping unit 20b. For example, the partition wall 20j1 is formed by using resin such as plastic. Further, for example, the thermal insulation member 20j2 is formed by using a fiber-based thermal insulation material or a foam plastic-based thermal insulation material.

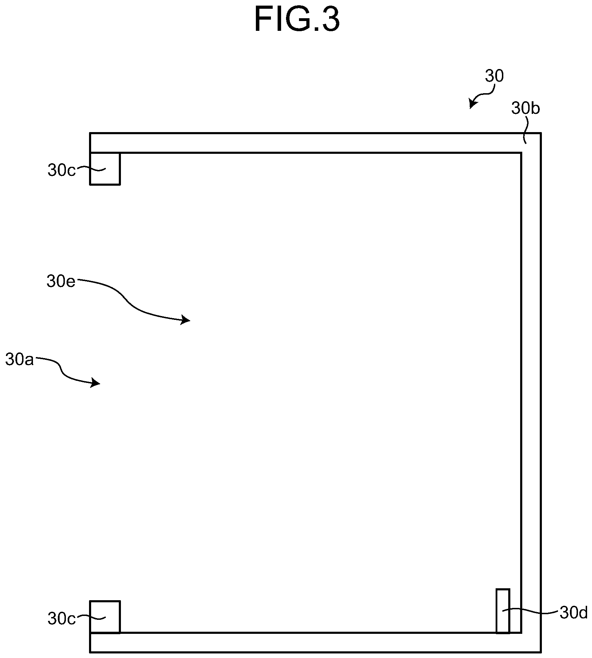

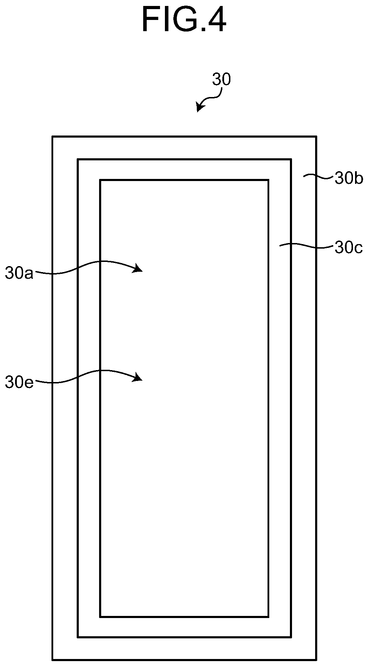

Next, an example of the incubator 30 according to the first embodiment will be explained. FIG. 3 is a diagram illustrating an example of an internal structure of the incubator 30 according to the first embodiment. FIG. 4 is a diagram illustrating an example of the incubator 30 according to the first embodiment, as viewed from the side of a slot 30a (hereinafter, "the slot 30a side"). FIG. 5 is a cross-sectional view of the incubator 30 according to the first embodiment taken on a plane going through an observation window 30f, illustrating an example of the incubator 30 as viewed from the side opposite from the slot 30a side.

As illustrated in FIGS. 3 to 5, the incubator 30 includes a casing 30b, a seal structure (a seal member) 30c, and a regulating member (a stopper) 30d.

The casing 30b has a substantially cuboid shape being hollow inside and has the slot 30a formed in one of the faces thereof. The slot 30a is an opening part being a boundary part between an internal space 30e formed by the casing 30b and the outside of the incubator 30. With this configuration, the incubator 30 has the internal space 30e and is capable of adjusting one or both of temperature and humidity of gas in the internal space 30e. In the present embodiment, instead of the entirety of the cartridge 20, a part of the cartridge 20 is inserted into the internal space 30e.

Further, provided in the bottom face of the casing 30b is the observation window 30f formed by using a transparent member such as a glass member.

The seal structure 30c is a member configured, while in a state where a part of the cartridge 20 is inserted in the internal space 30e, to prevent the gas in the internal space 30e from leaking to the outside. The regulating member 30d is a member configured to regulate movements of the cartridge 20 in the insertion direction of the cartridge 20. The seal structure 30c and the regulating member 30d will be explained later.

The observation apparatus 40 is an apparatus used by the user 90 at the time of observing the inside of the culturing container 20f through the observation window 30f, while in the state where a part of the cartridge 20 is inserted in the incubator 30. For example, the observation apparatus 40 may be one of various types of microscopes, or the like.

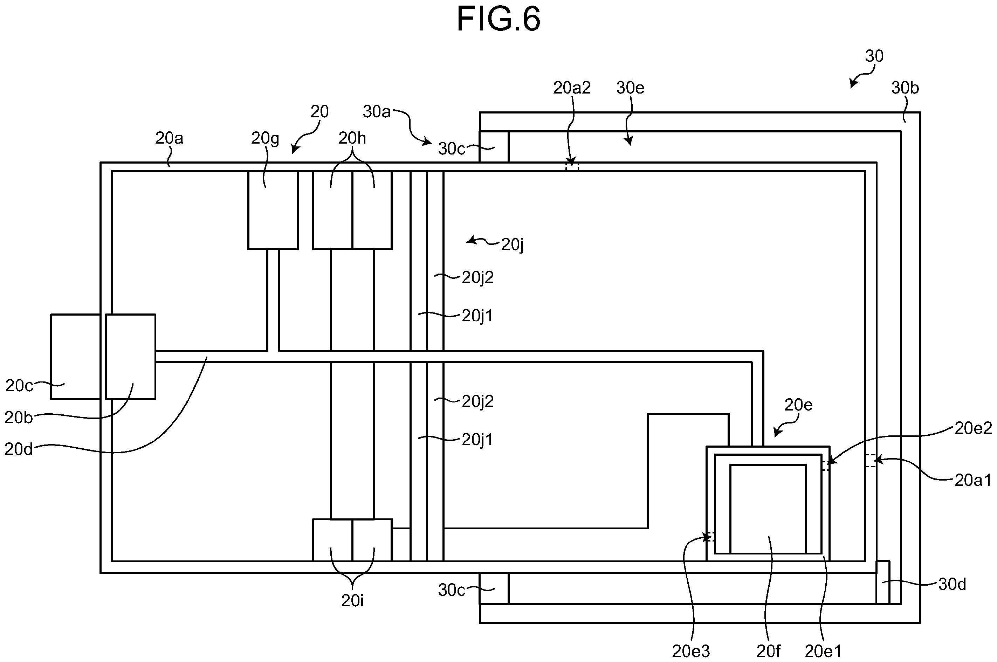

Next, an example of the state of the culturing system 1 where a part of the cartridge 20 is inserted in the incubator 30 will be explained. FIG. 6 is a diagram illustrating an example of an internal structure of the culturing system 1 in the state where a part of the cartridge 20 is inserted in the incubator 30 according to the first embodiment.

As illustrated in FIG. 6, while in the state where a part of the cartridge 20 is inserted in the incubator 30, gas adjusted by the incubator 30 so that one or both of the temperature and the humidity within the internal space 30e are suitable for the cell culturing is supplied to the internal space of the cartridge 20 through the gas supply port 20a1. The gas that has been supplied to the internal space of the cartridge 20 is further supplied to the culturing container 20f through the gas supply port 20e2 of the culturing unit 20e. In this manner, in the culturing system 1, as a result of the incubator 30 supplying the gas suitable for the cell culturing to the culturing container 20f provided in the cartridge 20, the culturing container 20f is exposed to the gas suitable for the culturing, so that the cells in the culturing container 20f are cultured.

Further, the gas that has been supplied to the culturing container 20f is discharged into an internal space of the cartridge 20 through the gas discharge port 20e3 of the culturing unit 20e. After that, the gas in the internal space of the cartridge 20 is discharged into the internal space 30e of the incubator 30 through the gas discharge port 20a2. Further, the gas in the internal space 30e is adjusted, again, by the incubator so as to bring one or both of the temperature and the humidity into a state suitable for the cell culturing. Furthermore, the gas in the internal space 30e re-adjusted in this manner is supplied, again, to the internal space of the cartridge 20 through the gas supply port 20a1. The gas in the internal space of the cartridge 20 may directly be supplied to the culturing unit 20e, by connecting the gas supply port 20a1 to the gas supply port 20e2, without going through the internal space of the cartridge 20. Similarly, exhaust from the culturing container 20f may also directly be discharged into the internal space 30e by connecting the gas discharge port 20e3 to the gas discharge port 20a2. As explained herein, in the culturing system 1, the gas is circulated between the internal space 30e of the incubator 30 and the culturing container 20f, while being adjusted so as to be in the state suitable for culturing the cells. Consequently, by using the culturing system 1 according to the present embodiment, it is possible to keep the culturing container 20f exposed to the gas suitable for the cell culturing at all times, while in the state where a part of the cartridge 20 is inserted in the incubator 30.

Further, the seal structure 30c is formed by using an elastic body such as a rubber gasket and is provided along the entirety of an inner surface of the abovementioned opening part of the casing 30b. As illustrated in FIG. 6, while in the state where a part of the cartridge 20 is inserted in the internal space 30e, the seal structure 30c is in close contact with the casing 20a of the of the cartridge 20. With this configuration, it is possible to prevent the gas in the internal space 30e from leaking to the outside. As a result, the incubator 30 is able to easily adjust the gas in the internal space 30e.

Further, at the time of inserting the cartridge 20 into the internal space 30e, to avoid obstructing the insertion of the cartridge 20 into the internal space 30e, the seal structure 30c is structured in such a manner that the seal structure 30c is expanded by a member (not illustrated) so that the space formed by the seal structure 30c is enlarged in size. After that, when the movement of the cartridge 20 is stopped as a result of the cartridge 20 abutting against the regulating member 30d as explained later, the expansion of the seal structure 30c is cancelled so that the seal structure 30c comes in close contact with the casing 20a of the cartridge 20.

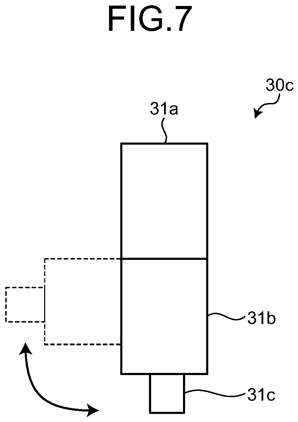

FIG. 7 is a diagram illustrating an exemplary configuration of the seal structure 30c according to the first embodiment. For example, as illustrated in FIG. 7, the seal structure 30c includes a driving unit (an actuator) 31a, a main body 31b, and an elastic body 31c. The driving unit 31a is an air cylinder, a robot, or the like and is configured to turn the main body 31b as indicated by the bidirectional arrow in FIG. 7. The main body 31b is a substantially cuboid member formed by using metal such as stainless steel or aluminum and is provided as to be turnable the driving unit 31a. Provided on the bottom face of the main body 31b is the elastic body 31c. The elastic body 31c is formed by using a rubber composite material having high thermal conductivity or a sheet having high thermal conductivity.

At the time of inserting the cartridge 20 into the internal space 30e, the driving unit 31a is configured to turn the main body 31b to the position indicated with the dotted lines, so as to avoid obstructing the insertion of the cartridge 20 into the internal space 30e. Further, when the movement of the cartridge 20 is stopped as a result of the cartridge 20 abutting against the regulating member 30d as explained later, the driving unit 31a is configured to turn the main body 31b to the position indicated by the solid line. As a result, the elastic body 31c comes into close contact with the casing 20a of the cartridge 20.

Further, as illustrated in FIG. 6, while in the state where a part of the cartridge 20 is inserted in the internal space 30e, the thermal insulation unit 20j is configured to prevent the heat in the space in which the culturing container 20f is present from being conducted to the space in which the culture medium keeping unit 20b is present. With this configuration, it is possible to prevent the space in which the culture medium keeping unit 20b is present from being warmed. As a result, the culture medium keeping unit 20b is able to keep the culture medium at a temperature suitable for keeping the culture medium. In addition, the cartridge 20 is able to deliver the culture medium kept at a temperature suitable for keeping the culture medium, to the culturing container 20f.

Further, as illustrated in FIG. 6, the cartridge 20 is inserted into the incubator 30, while in the state of abutting against the regulating member 30d. In this situation, the regulating member 30d is configured to regulate movements in the insertion direction of the cartridge 20, so that the thermal insulation unit 20j of the cartridge 20 is positioned more outward than the seal structure 30c while the cartridge 20 is inserted in the internal space 30e. With this configuration, it is possible to prevent the space in which the culture medium keeping unit 20b is present from being warmed by the gas in the internal space 30e of the incubator 30. As a result, the culture medium keeping unit 20b is further able to keep the culture medium at a temperature suitable for keeping the culture medium. In addition, the cartridge 20 is further able to deliver the culture medium kept at a temperature suitable for keeping the culture medium, to the culturing container 20f.

Furthermore, with the above configurations, the culturing system 1 according to the present embodiment is able to prevent the occurrence of contamination at the time of replacing the culture medium in the culturing container 20f. Consequently, the culturing system 1 according to the present embodiment is able to culture the cells with high quality.

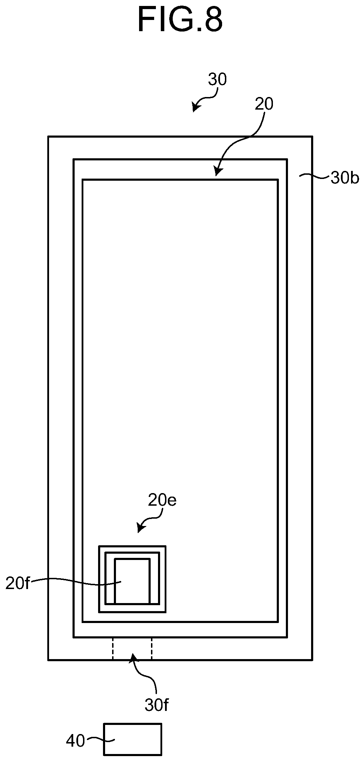

FIG. 8 is a cross-sectional view of an example of the culturing system 1 taken on a plane going through the observation window 30f, in the state where a part of the cartridge 20 is inserted in the incubator 30 according to the first embodiment, illustrating an example of the incubator 30 as viewed from the side opposite from the slot 30a side. As illustrated in FIG. 8, while in the state where a part of the cartridge 20 is inserted in the incubator 30, the user 90 is able to observe the inside of the culturing container 20f through the observation window 30f, by using the observation apparatus 40.

The culturing system 1 according to the first embodiment has thus been explained. By using the culturing system 1 according to the first embodiment, it is possible to culture the cells with high quality, as explained above.

### Various Modification Examples

Next, various modification examples of the culturing system 1 according to the first embodiment will be explained. The following description of the modification examples will primarily explain differences from the culturing system 1 according to the first embodiment described above, and explanations of the same configurations may be omitted. The following will describe first to fifth modification examples. First Modification Example

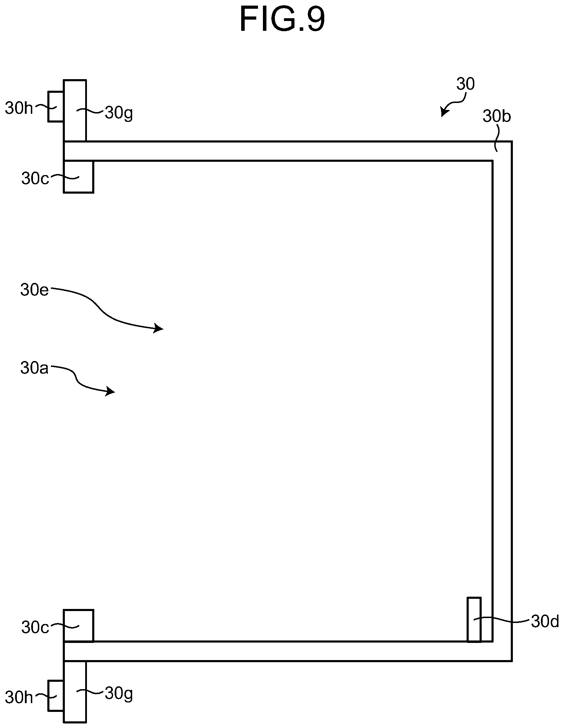

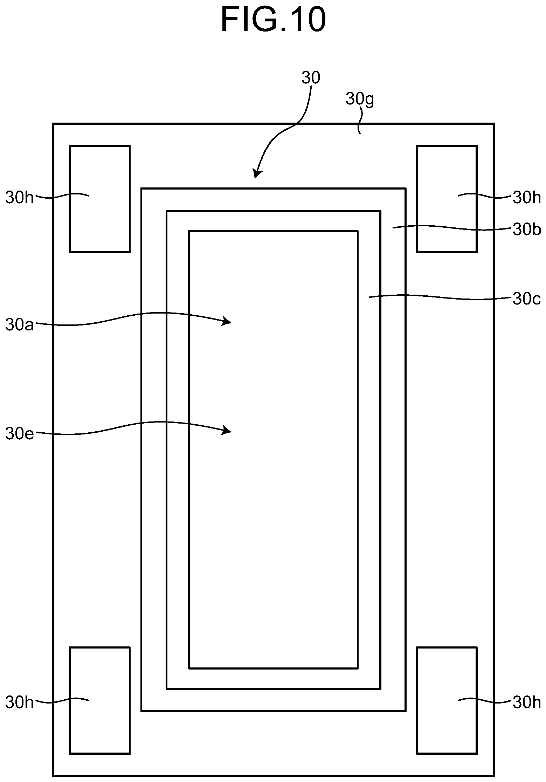

FIG. 9 is a diagram illustrating an example of an internal structure of the incubator 30 in the culturing system 1 according to the first modification example. FIG. 10 is a diagram illustrating an example of the incubator 30 according to the first modification example, as viewed from the slot 30a side.

As illustrated in FIGS. 9 and 10, the incubator 30 according to the first modification example includes, at the opening part described above, a flange part 30g and heating structures 30h. The flange part 30g is formed by using a member having a thermal insulation structure. The heating structures 30h are realized by using heating mechanisms, for example, and are configured to heat certain members and gas positioned in the surroundings of the heating structures 30h. For example, the heating structures 30h are configured to heat the opening part of the casing 30b of the incubator 30. In this regard, if the incubator 30 did not include the flange part 30g and the heating structures 30h, the opening part of the casing 30b of the incubator 30 would be cooled by the gas on the outside of the incubator 30. In that situation, there would be a possibility that condensation might occur on the inner surface of the opening part of the casing 30b of the incubator 30.

In contrast, in the first modification example, the incubator 30 includes, at the opening part of the casing 30b, the flange part 30g having the thermal insulation structure and the heating structures 30h. Accordingly, it is possible to prevent the opening part of the casing 30b of the incubator 30 from being cooled by the gas on the outside of the incubator 30. As a result, the culturing system 1 according to the first modification example is able to prevent the occurrence of the condensation on the inner surface of the opening part of the casing 30b of the incubator 30.

Alternatively, at the opening part of the casing 30b, the incubator 30 may include only the thermal insulation structure (the flange part 30g) or may include only the heating structures 30h. In other words, it is sufficient when the incubator 30 includes at least one selected from between the thermal insulation structure and the heating structures 30h, at the opening part of the casing 30b. Second Modification Example

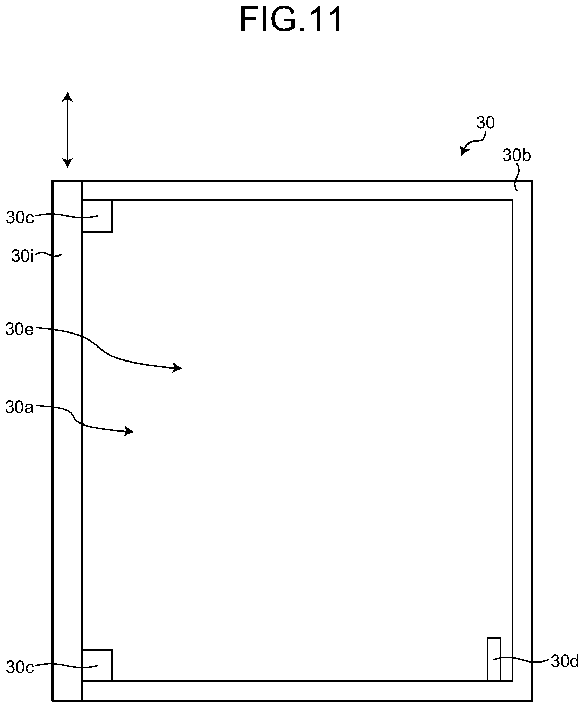

FIG. 11 is a diagram illustrating an example of an internal structure of the incubator 30 in the culturing system 1 according to a second modification example. As illustrated in FIG. 11, the incubator 30 according to the second modification example includes a shutter 30i capable of covering the opening of the opening part of the casing 30b, over an area larger than the opening. The shutter 30i is provided so as to be movable in the up-and-down directions indicated by the bidirectional arrow in FIG. 11. While the cartridge 20 is not inserted in the internal space 30e, the shutter 30i is configured to move to a position (a close position) where it is possible to close the opening of the opening part of the casing 30b. In contrast, when the cartridge 20 is to be inserted into the internal space 30e and the like, the shutter 30i is configured to move to a position (an open position) where it is possible to open the opening of the opening part of the casing 30b. For example, as the open position, the shutter 30i may move to a position above the close position. The shutter 30i may manually be moved by the user 90 or may automatically be moved by a driving mechanism such as an air cylinder.

As explained above, in the second modification example, while the cartridge 20 is not inserted in the internal space 30e, the shutter 30i is configured to close the opening of the opening part of the casing 30b. With this configuration, it is possible to prevent the gas in the internal space 30e from leaking to the outside. As a result, the incubator 30 is able to easily adjust the gas in the internal space 30e. Third Modification Example

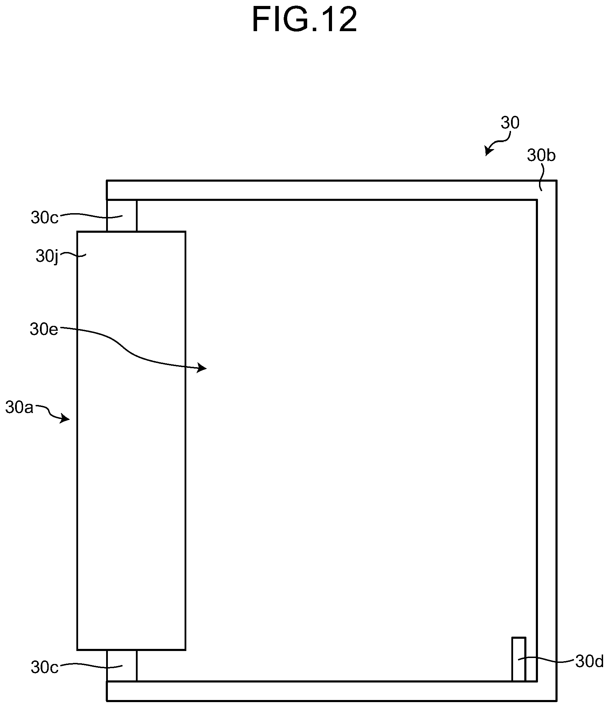

FIG. 12 is a diagram illustrating an example of an internal structure of the incubator 30 in the culturing system 1 according to a third modification example. As illustrated in FIG. 12, the incubator 30 according to the third modification example includes a mock cartridge 30j. The mock cartridge 30j is a substantially cuboidal member. The mock cartridge 30j is a member configured to close the opening of the opening part of the casing 30b while the cartridge 20 is not inserted in the internal space 30e. For example, as a result of a part of the mock cartridge 30j being inserted in the internal space 30e of the incubator 30, the opening of the opening part of the casing 30b is closed.

As explained above, in the third modification example, while the cartridge 20 is not inserted in the internal space 30e, the mock cartridge 30j is configured to close the opening of the opening part of the casing 30b. With this configuration, similarly to the first modification example, it is possible to prevent the gas in the internal space 30e from leaking to the outside and to easily adjust the gas in the internal space 30e.

### Fourth Modification Example

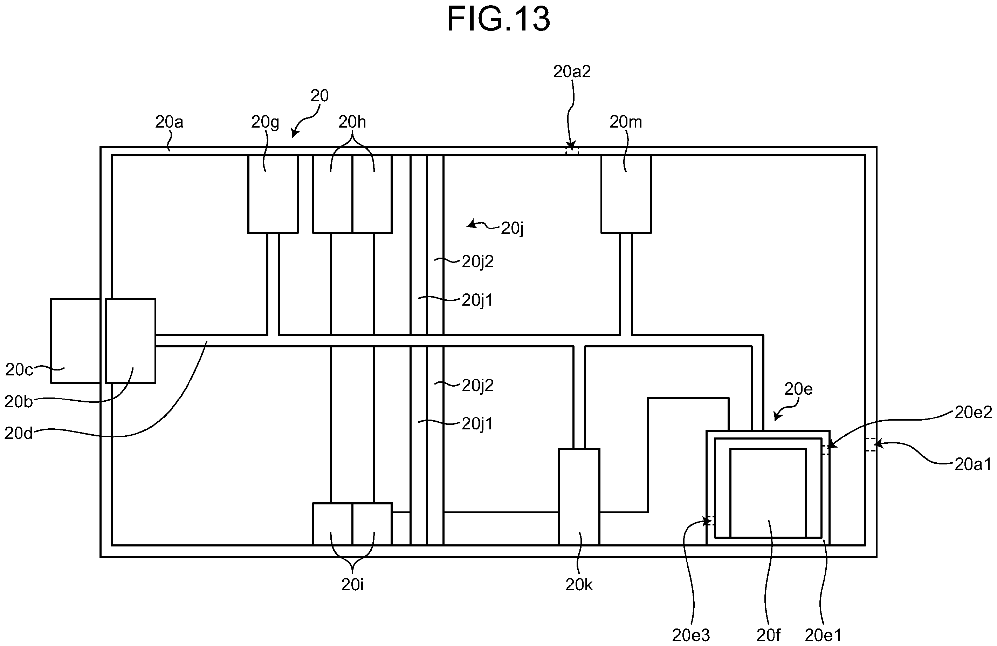

FIG. 13 is a diagram illustrating an example of an internal structure of the cartridge 20 according to a fourth modification example. FIG. 13 is a front view of the cartridge 20, as seen through the front face of the casing 20a of the cartridge 20. As illustrated in FIG. 13, the cartridge 20 according to the fourth modification example includes a storage unit 20k and a liquid delivery unit 20m, in addition to the plurality of constituent elements included in the cartridge 20 according to the first embodiment described above.

The storage unit 20k is provided in the space in which the culturing container 20f is present and connected to the flow path 20d. In the fourth modification example, the culture medium flowing through the flow path 20d is delivered to the culturing container 20f provided in the culturing unit 20e via the storage unit 20k, instead of being directly delivered to the culturing container 20f provided in the culturing unit 20e.

For example, the culture medium flowing through the flow path 20d is temporarily stored in the storage unit 20k. In this situation, the culture medium stored in the storage unit 20k is the culture medium that has been kept cold in the culture medium keeping unit 20b, and the culture medium therefore has a relatively low temperature. For this reason, the culture medium is to be stored in the storage unit 20k for a prescribed time period, until the temperature of the culture medium stored in the storage unit 20k reaches a temperature suitable for the cell culturing performed by the culturing container 20f. The prescribed time period is a time period calculated through an experiment in advance and denotes the time period from when the culture medium is stored into the storage unit 20k, to when the temperature of the culture medium stored in the storage unit 20k reaches the temperature suitable for the cell culturing.

The liquid delivery unit 20m is connected to the flow path 20d and is configured to suck, from the storage unit 20k, the culture medium that has been stored in the storage unit 20k for the prescribed time period. After that, the liquid delivery unit 20m is configured to deliver the sucked culture medium to the culturing container 20f provided in the culturing unit 20e via the flow path 20d. For example, the liquid delivery unit 20m may have the same configuration as that of the liquid delivery unit 20g.

As explained above, in the fourth modification example, the flow path 20d is provided with the storage unit 20k used for warming the culture medium that has been kept cold in the culture medium keeping unit 20b. Thus, the culture medium at the temperature suitable for the cell culturing is delivered to the culturing container 20f. Consequently, the culturing system 1 according to the fourth modification example is able to culture the cells with higher quality.

### Fifth Modification Example

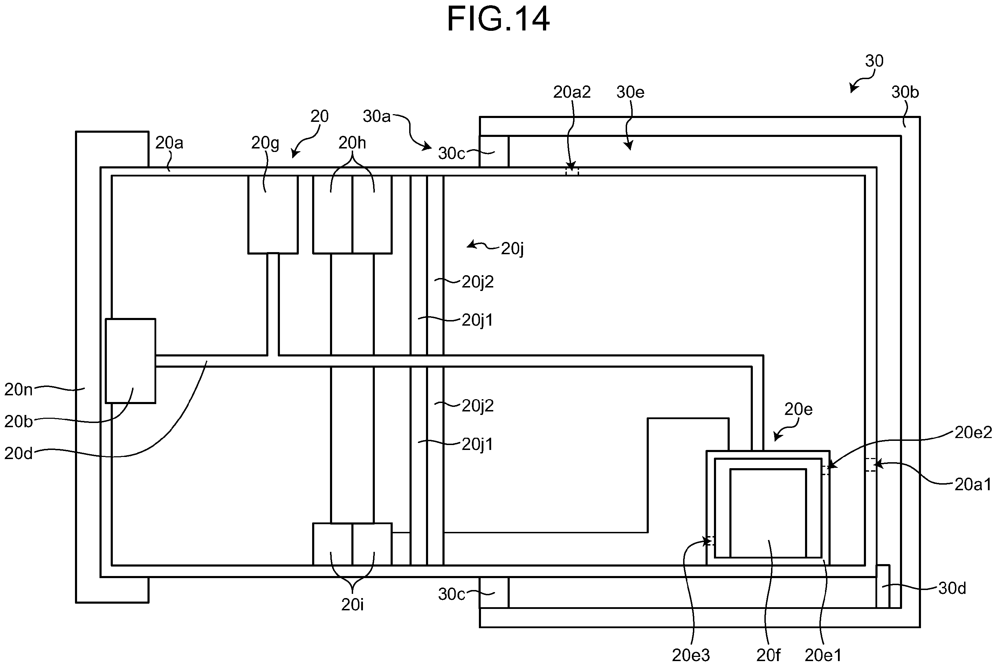

FIG. 14 is a diagram illustrating an example of an internal structure of the cartridge 20 according to the fifth modification example. FIG. 14 is a front view of the cartridge 20, as seen through the front face of the casing 20a of the cartridge 20. As illustrated in FIG. 14, the culturing system 1 according to the fifth modification example includes a cold storage apparatus 20n. However, the cartridge 20 according to the fifth modification example does not include the cold storage unit 20c included in the cartridge 20 according to the first embodiment described above.

The cold storage apparatus 20n may be a cooler or the like, for example. In the fifth modification example, while in the state where a part of the cartridge 20 is inserted in the internal space 30e, the cold storage apparatus 20n is configured to keep cool the culture medium kept in the culture medium keeping unit 20b, while the cartridge 20 is inserted in the cold storage apparatus 20n. The cold storage apparatus 20n is provided in a position where it is possible to keep cool the culture medium kept in the culture medium keeping unit 20b, while in the state where a part of the cartridge 20 is inserted in the internal space 30e. The cold storage apparatus 20n is configured to keep the culture medium cool, so that the culture medium kept in the culture medium keeping unit 20b is kept while remaining at a temperature (e.g., 5 degrees centigrade) suitable for keeping the culture medium.

Consequently, similarly to the cold storage unit 20c, the cold storage apparatus 20n is able to have the culture medium kept in the culture medium keeping unit 20b at the temperature suitable for keeping the culture medium.

### Second Embodiment

In the first embodiment described above, due to a temperature difference between the internal space 30e and external ambient air, condensation may occur on the internal space 30e side of the seal structure 30c at the opening part of the casing 30b being a boundary between the internal space 30e of the incubator 30 and the external ambient air. To culture the cells with high quality, it is necessary to keep temperature, humidity, and a gas composition constant. When the condensation occurs at the opening part, a problem may arise where the humidity in the incubator 30 fluctuates, and the quality of the culturing becomes unstable. Further, another problem may also arise where cleaning needs to be carried out more frequently to avoid growth of mold and bacteria.

To cope with the circumstances described above, a culturing system capable of preventing the occurrence of the condensation on the internal space side of the seal structure of the incubator will be described as a culturing system according to a second embodiment. In the description of the second embodiment, some of the constituent elements that are the same as those in the first embodiment will be referred to by using the same reference characters as in the first embodiment, and explanations of those constituent elements may be omitted. Further, as long as no conflict occurs, the culturing system according to the second embodiment may include any of the configurations in the first to the fifth modification examples described above.

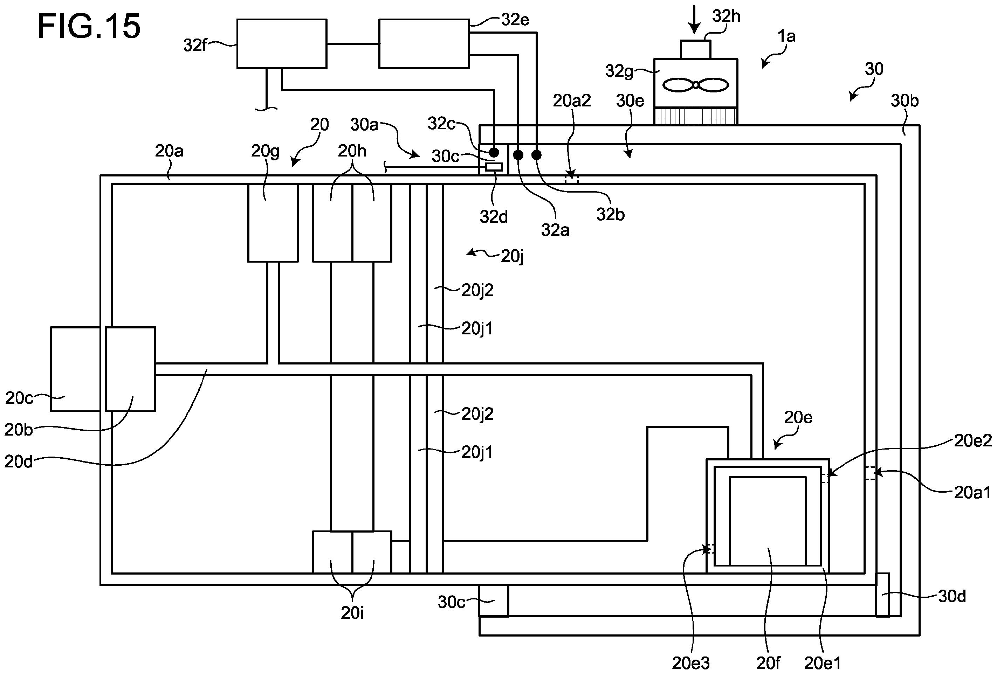

FIG. 15 is a diagram illustrating an exemplary configuration of a culturing system 1a according to the second embodiment. As compared to the culturing system 1 illustrated in FIG. 6, the culturing system 1a illustrated in FIG. 15 is different from the culturing system 1 illustrated in FIG. 6 for including a first thermometer 32a, a hygrometer 32b, a second thermometer 32c, a heater 32d, calculating circuitry 32e, controlling circuitry 32f, a fan 32g, and a gas introduction port 32h.

The first thermometer 32a is configured to measure a temperature T1 of the internal space 30e at prescribed time intervals. More specifically, the first thermometer 32a is configured to measure the temperature T1 of the gas in the internal space 30e. Further, every time a temperature T1 is measured, the first thermometer 32a is configured to transmit a signal (a temperature T1 signal) indicating the temperature T1 to the calculating circuitry 32e. More specifically, for example, the first thermometer 32a is configured to measure, within the internal space 30e, the temperature T1 of a space in the vicinity of the seal structure 30c. For example, the first thermometer 32a is provided in a position within the internal space 30e, while being positioned apart from the seal structure 30c by a distance in the range from 35 millimeters or more to 100 millimeters or less inclusive and is configured to measure the temperature T1 of the space that is within the internal space 30e and is positioned apart from the seal structure 30c by a distance in the range from 35 millimeters or more to 100 millimeters or less inclusive. Further, the first thermometer 32a is provided in a position closer to the seal structure 30c than the hygrometer 32b is. The temperature T1 is an example of the first temperature.

The hygrometer 32b is configured to measure a relative humidity RH of the internal space 30e at prescribed time intervals. More specifically, the hygrometer 32b is configured to measure the relative humidity RH of the gas in the internal space 30e. Further, every time a relative humidity RH is measured, the hygrometer 32b is configured to transmit a signal (a humidity signal) indicating the relative humidity RH to the calculating circuitry 32e. More specifically, the hygrometer 32b is configured to measure, within the internal space 30e, the relative humidity RH of a space in the vicinity of the seal structure 30c. For example, the hygrometer 32b is provided in a position within the internal space 30e while being positioned apart from the seal structure 30c by a distance in the range from 35 millimeters or more to 100 millimeters or less inclusive and is configured to measure the relative humidity RH of the space that is within the internal space 30e and is positioned apart from the seal structure 30c by a distance in the range from 35 millimeters or more to 100 millimeters or less inclusive. The relative humidity RH is an example of the humidity.

The second thermometer 32c is configured to measure a temperature T2 of the seal structure 30c at prescribed time intervals. Further, every time a temperature T2 is measured, the second thermometer 32c is configured to transmit a signal (a temperature T2 signal) indicating the temperature T2 to the controlling circuitry 32f. For example, the second thermometer 32c is provided for the main body 31b of the seal structure 30c and is configured to measure the temperature T2 of the main body 31b. In another example, in the state where the cartridge 20 is inserted in the internal space 30e, the second thermometer 32c may be provided in the vicinity of a part (an abutment part) of the cartridge 20 where the cartridge 20 abuts against the seal structure 30c. For example, the second thermometer 32c may be provided in a position apart from the abutment part by a distance in the range from 10 millimeters or more to 20 millimeters or less inclusive and configured to measure the temperature T2 in that position (the temperature of the seal structure 30c). The temperature T2 is an example of the second temperature.

The heater 32d may be a cartridge heating mechanism, a silicone rubber corded heating mechanism, a heated water circulator, or the like and is provided for the seal structure 30c. The heater 32d is configured to adjust the temperature of the seal structure 30c by heating the seal structure 30c.

At prescribed time intervals, the calculating circuitry 32e is configured to calculate a dew point DPT of the internal space 30e, on the basis of the temperature T1 indicated by the temperature T1 signal transmitted from the first thermometer 32a and the relative humidity RH indicated by the humidity signal transmitted from the hygrometer 32b. For example, the calculating circuitry 32e is configured to calculate the dew point DPT from the most recent temperature T1 and the most recent relative humidity RH. More specifically, on the basis of the temperature T1 and the relative humidity RH, the calculating circuitry 32e is configured to calculate water vapor pressure and to calculate a temperature at which the water vapor pressure corresponds to saturation water vapor pressure as the dew point DPT. Further, every time a dew point DPT is calculated, the calculating circuitry 32e is configured to transmit information (dew point information) indicating the dew point DPT to the controlling circuitry 32f. In other words, the calculating circuitry 32e is configured to calculate the dew point DPT of the internal space 30e, on the basis of the temperature T1 measured by the first thermometer 32a and the relative humidity RH measure by the hygrometer 32b. The calculating circuitry 32e is realized by using one or more processors, for example. The calculating circuitry 32e is an example of the calculating unit.

At prescribed time intervals, the controlling circuitry 32f is configured to control the heating temperature of the heater 32d for the seal structure 30c, on the basis of the dew point DPT indicated by the dew point information. More specifically, for example, the controlling circuitry 32f is configured to receive the dew point information and the temperature T2 signal at prescribed time intervals. Further, every time dew point information and a temperature T2 signal are received, the controlling circuitry 32f is configured to control the heating temperature of the heater 32d for the seal structure 30c, in such a manner that the temperature T2 indicated by the received temperature T2 signal becomes equal to or higher than the dew point DPT indicated by the received dew point information. In other words, the controlling circuitry 32f is configured to control the heater 32d so that the temperature T2 measured by the second thermometer 32c becomes equal to or higher than the dew point DPT. As a result, the temperature of the seal structure 30c becomes equal to or higher than the dew point DPT. Consequently, by using the culturing system 1a according to the second embodiment, it is possible to prevent the occurrence of the condensation on the internal space 30e side of the seal structure 30c.

In an example, the first thermometer 32a described above may be configured, every time a temperature T1 is measured, to transmit the temperature T1 signal also to the controlling circuitry 32f, in addition to the calculating circuitry 32e. Further, every time a temperature T1 signal, dew point information, and a temperature T2 signal are received, the controlling circuitry 32f may be configured to control the heating temperature of the heater 32d for the seal structure 30c so that the temperature T2 indicated by the received temperature T2 signal becomes equal to or higher than the dew point DPT indicated by the received dew point information and equal to or lower than the temperature T1 indicated by the received temperature T1 signal.

In other words, the controlling circuitry 32f may be configured to control the heater 32d, so that the temperature T2 measured by the second thermometer 32c becomes equal to or higher than the dew point DPT and equal to or lower than the temperature T1 measured by the first thermometer 32a. With this configuration, the temperature of the seal structure 30c is a temperature in the range from the dew point DPT to the temperature T1 inclusive. Consequently, by using the culturing system 1a according to the second embodiment, it is possible to prevent the temperature inside the internal space 30e from becoming a high temperature that may impose an adverse impact on the cell culturing, while preventing the occurrence of the condensation on the internal space 30e side of the seal structure 30c.

The controlling circuitry 32f is an example of the controlling unit. The controlling circuitry 32f is realized by using one or more processors.

Further, the processes performed by the calculating circuitry 32e and the controlling circuitry 32f may be performed by a single piece of processing circuitry in place of the calculating circuitry 32e and the controlling circuitry 32f. The processing circuitry is realized by one or more processors.

The fan 32g is provided inside the internal space 30e, is provided with a suction port through which the gas is sucked from the inside of the internal space 30e and a blow-out port through which the sucked gas is blown out, and is configured to circulate the gas in the internal space 30e. With this configuration, the fan 32g is able to inhibit unevenness in the temperature and unevenness in the humidity inside the internal space 30e. As a result, the gas of which unevenness in the temperature and unevenness in the humidity are inhibited is to be supplied to the culturing container 20f provided in the cartridge 20.

The gas introduction port 32h is an introduction port used for introducing the gas of which one or both of temperature and humidity have been adjusted.

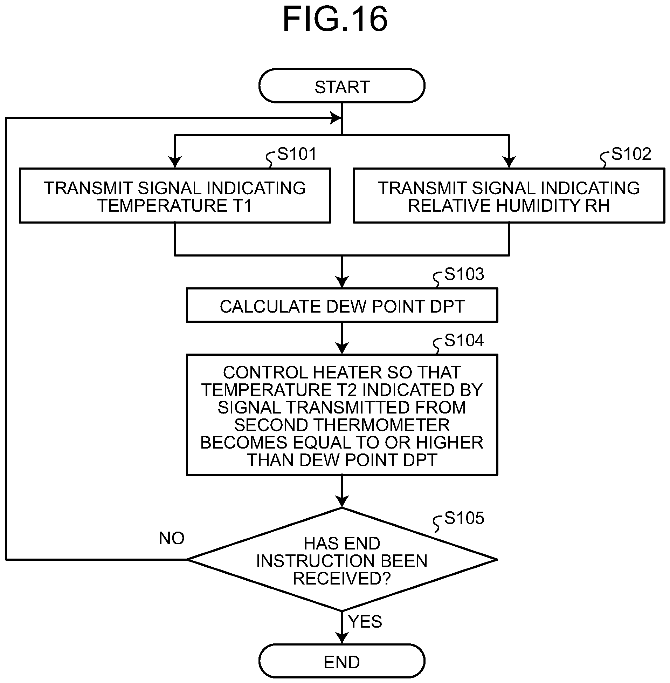

FIG. 16 is a flowchart illustrating a flow in a temperature controlling process executed by the culturing system 1a according to the second embodiment. The temperature controlling process illustrated in FIG. 16 is executed when the user inputs, to the culturing system 1a, an instruction to execute the temperature controlling process, via an input interface (not illustrated) realized with a mouse, a keyboard, and/or the like.

As illustrated in FIG. 16, to begin with, the process at step S101 and the process at step S102 are executed in parallel. More specifically, the first thermometer 32a measures a temperature T1 of the internal space 30e and transmits a signal (the temperature T1 signal) indicating the temperature T1 to the calculating circuitry 32e (step S101). Also, the hygrometer 32b measures a relative humidity HR of the internal space 30e and transmits a signal (the humidity signal) indicating the relative humidity RH to the calculating circuitry 32e (step S102).

Subsequently, on the basis of the temperature T1 indicated by the temperature T1 signal and the relative humidity RH indicated by the humidity signal, the calculating circuitry 32e calculates a dew point DPT of the internal space 30e and transmits the dew point information to the controlling circuitry 32f (step S103).

After that, the controlling circuitry 32f controls the heating temperature of the heater 32d for the seal structure 30c so that the temperature T2 indicated by the temperature T2 signal becomes equal to or higher than the dew point DPT indicated by the dew point information (step S104).

Subsequently, the controlling circuitry 32f judges whether or not an instruction (an end instruction) to end the temperature controlling process being input by the user via the input interface has been received (step S105). When the end instruction has not been received (step S105: No), the controlling circuitry 32f returns to steps S101 and S102. Further, the culturing system 1a performs the processes at steps S101 through S105 again. The culturing system 1a performs the processes at steps S101 through S105 at prescribed time intervals, until it is determined at step S105 that the end instruction is received.

On the contrary, when the end instruction has been received (step S105: Yes), the controlling circuitry 32f ends the temperature controlling process.

The culturing system 1a according to the second embodiment has thus been explained. As described above, by using the culturing system 1a according to the second embodiment, it is possible to prevent the occurrence of the condensation on the internal space 30e side of the seal structure 30c.

Further, in the second embodiment, the first thermometer 32a may be provided in a position within the internal space 30e while being positioned in the vicinity of the gas introduction port 32h. For example, the first thermometer 32a may be provided in a position apart from the gas introduction port 32h by a distance in the range from 30 millimeters or more to 80 millimeters or less inclusive. When being provided in the vicinity of the gas introduction port 32h, the first thermometer 32a is able to measure the temperature T1 of fresh gas in the internal space 30e.

Alternatively, the first thermometer 32a may be provided in a position within the internal space 30e while being positioned in the vicinity of the blow-out port of the fan 32g. For example, the first thermometer 32a may be provided in a position apart from the blow-out port of the fan 32g by a distance within the range from 30 millimeters or more to 80 millimeters or less inclusive. When being provided in the vicinity of the blow-out ort of the fan 32g, the first thermometer 32a is able to measure the temperature T1 of the gas of which the temperature may easily change because of flowing fast in the internal space 30e.

Further, the hygrometer 32b may be provided in a position within the internal space 30e while being positioned in the vicinity of the gas introduction port 32h. For example, the hygrometer 32b may be provided in a position apart from the gas introduction port 32h by a distance in the range from 30 millimeters or more to 80 millimeters or less inclusive. When being provided in the vicinity of the gas introduction port 32h, the hygrometer 32b is able to measure the relative humidity RH of fresh gas in the internal space 30e.

In another example, the hygrometer 32b may be provided in a position within the internal space 30e while being positioned in the vicinity of the blow-out port of the fan 32g. For example, the hygrometer 32b may be provided in a position apart from the blow-out port of the fan 32g by a distance in the range from 30 millimeters or more to 80 millimeters or less inclusive. When being provided in the vicinity of the blow-out port of the fan 32g, the hygrometer 32b is able to measure the relative humidity RH of the gas of which the temperature may easily change because of flowing fast in the internal space 30e.

### Modification Example of Second Embodiment

There is a possibility that the seal structure 30c may partially be impacted by disturbance from external environment. For example, air blown from an air conditioner provided in the external environment may impact the temperature of the seal structure 30c, or heat discharged from a peripheral unit may impact the temperature of the seal structure 30c. To cope with these situations, the culturing system may include a plurality of seal structures in place of the single seal structure 30c, while each of the seal structures has the same configuration as described above, so that, with respect to each of the seal structures, the temperature of the seal structure is controlled. Thus, the culturing system configured in this manner will be explained as a culturing system according to a modification example of the second embodiment.

In the description of the modification example of the second embodiment, some of the constituent elements that are the same as those in the second embodiment will be referred to by using the same reference characters as in the second embodiment, and explanations of those constituent elements may be omitted. Further, as long as no conflict occurs, the culturing system according to the modification example of the second embodiment may include any of the configurations in the first to the fifth modification examples described above.

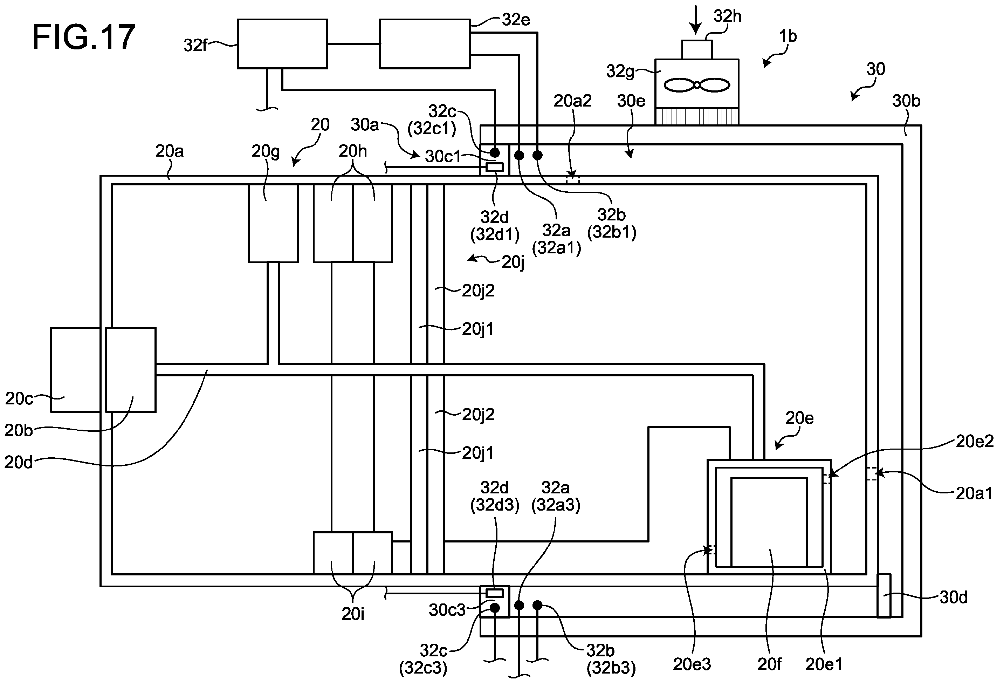

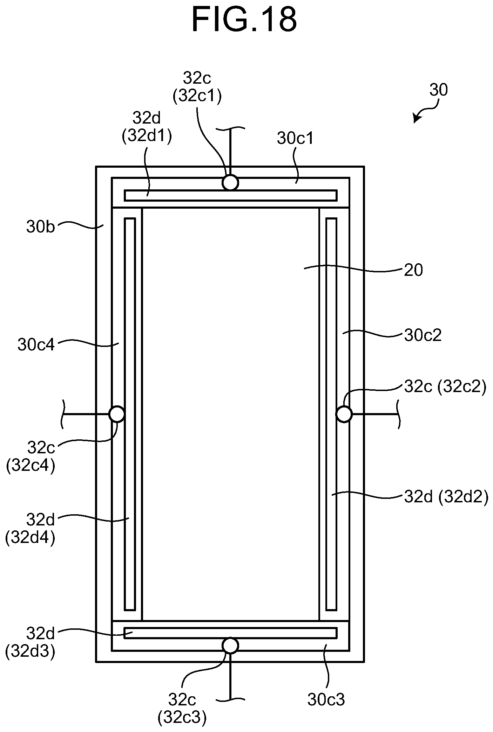

FIG. 17 is a diagram illustrating an exemplary configuration of a culturing system 1b according to the modification example of the second embodiment. FIG. 18 is a diagram illustrating an example of the incubator 30 according to the modification example of the second embodiment, as viewed from the slot 30a side. As compared with the culturing system 1a illustrated in FIG. 15, the culturing system 1b illustrated in FIGS. 17 and 18 is different from the culturing system 1a illustrated in FIG. 15 for including a plurality of seal structures 30c1 to 30c4. In the following description of the second embodiment, when not being distinguished from one another, the plurality of seal structures 30c1 to 30c4 may be referred to as "seal structures 30c".

Further, as compared with the culturing system 1a illustrated in FIG. 15, the culturing system 1b illustrated in FIGS. 17 and 18 is different from the culturing system 1a illustrated in FIG. 15, for including, with respect to each of the seal structures 30c, a first thermometer 32a, a hygrometer 32b, a second thermometer 32c, and a heater 32d, similarly to the second embodiment described above.

For example, in the example in FIG. 17, a first thermometer 32a1, a hygrometer 32b1, a second thermometer 32c1, and a heater 32d1 are provided in correspondence with the seal structure 30c1. Also, a first thermometer 32a3, a hygrometer 32b3, a second thermometer 32c3, and a heater 32d3 are provided in correspondence with the seal structure 30c3. The same applies to the seal structure 30c2 and the seal structure 30c4. In other words, in correspondence with the seal structure 30c2, a first thermometer 32a2 (not illustrated), a hygrometer 32b2 (not illustrated), a second thermometer 32c2, and a heater 32d2 are provided. Also, in correspondence with the seal structure 30c4, a first thermometer 32a4 (not illustrated), a hygrometer 32b4 (not illustrated), a second thermometer 32c4, and a heater 32d4 are provided.

Accordingly, the plurality of seal structures 30c1 to 30c4 correspond to a plurality of spaces included in the internal space 30e. For example, the seal structure 30c1 corresponds to a space being positioned within the internal space 30e and including the first thermometer 32a1 and the hygrometer 32b1. Similarly, the seal structure 30c3 corresponds to a space being positioned within the internal space 30e and including the first thermometer 32a3 and the hygrometer 32b3. The same applies to the seal structure 30c2 and the seal structure 30c4.

Further, in the modification example of the second embodiment, each of the plurality of first thermometers 32a1 to 32a4 is configured to measure a temperature T1 of a corresponding one of the plurality of spaces described above and to transmit a temperature T1 signal to the calculating circuitry 32e.

Also, each of the plurality of hygrometers 32b1 to 32b4 is configured to measure a relative humidity RH of a corresponding one of the plurality of spaces described above and to transmit a humidity signal to the calculating circuitry 32e.

Further, each of the plurality of second thermometers 32c1 to 32c4 is configured to measure a second temperature T2 of a corresponding one of the plurality of seal structures 30c1 to 30c4 and to transmit a temperature T2 signal to the controlling circuitry 32f.

In addition, each of the plurality of heaters 32d1 to 32d4 is configured to heat a corresponding one of the plurality of seal structures 30c1 to 30c4.

Furthermore, in the modification example of the second embodiment, the calculating circuitry 32e and the controlling circuitry 32f are configured, with respect to each of the seal structures 30c, to perform the same processes as explained above in the second embodiment.

In other words, with respect to each of the plurality of spaces described above, the calculating circuitry 32e is configured to calculate a dew point DPT on the basis of the measured temperature T1 and the measured relative humidity RH. Further, the controlling circuitry 32f is configured to control each of the plurality of heaters 32d1 to 32d4, so that the temperature T2 measured with respect to each of the plurality of seal structures 30c1 to 30c4 becomes equal to or higher than a corresponding one of the dew points DPT.

The culturing system 1b according to the modification example of the second embodiment has thus been explained. In the culturing system 1b according to the modification example of the second embodiment, with respect to each of the seal structures 30c, the temperature of the seal structure 30c is controlled. Consequently, it is possible to prevent, with higher certainty, the occurrence of the condensation on the internal space 30e side of each of the plurality of seal structures 30c1 to 30c4.

According to at least one aspect of the embodiments and the modification examples described above, it is possible to prevent the occurrence of condensation on the internal space side of the one or more seal structures.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A culturing system (1, 1a, 1b) comprising:
an incubator (30) that has an internal space (30e) and is capable of adjusting one or both of a temperature and a humidity of gas in the internal space (30e); and
a cartridge (20) capable of accommodating a culturing container (20f) used for culturing a cell, a culture medium keeping unit (20b) configured to keep therein a culture medium kept cold, and a flow path (20d) which connects the culturing container (20f) to the culture medium keeping unit (20b) and through which the culture medium kept in the culture medium keeping unit (20b) flows toward the culturing container (20f), wherein
the gas in the internal space (30e) is supplied to the culturing container (20f), by inserting a part of the cartridge (20) into the internal space (30e).

2. The culturing system (1, 1a, 1b) according to claim 1, wherein
the cartridge includes a thermal insulation unit (20j), and
the thermal insulation unit (20j) is provided between the culturing container (20f) and the culture medium keeping unit (20b) and is configured to isolate the culturing container (20f) from the culture medium keeping unit (20b), preferably, wherein the thermal insulation unit (20j) is formed while including a partition wall (20j1) and a thermal insulation member (20j2) configured to isolate the culturing container (20f) from the culture medium keeping unit (20b).

3. The culturing system (1, 1a, 1b) according to claim 1 or 2, wherein the incubator (30) includes a seal structure (30c) provided at an opening part (30a) being a boundary part between the internal space (30e) and an outside of the incubator (30).

4. The culturing system (1, 1a, 1b) according to any one of claims 1 to 3, wherein the incubator (30) includes one or both of a thermal insulation structure (30g) and a heating structure (30h) provided at an opening part (30a) being a boundary part between the internal space (30e) and an outside of the incubator (30).

5. The culturing system (1, 1a, 1b) according to any one of claims 1 to 4, wherein the incubator (30) includes a shutter (30i) configured, while the cartridge (20) is not inserted in the internal space (30e), to close an opening of an opening part (30a) being a boundary part between the internal space (30e) and an outside of the incubator (30).

6. The culturing system (1, 1a, 1b) according to any one of claims 1 to 4, wherein inserted into the incubator (30) is a member (30j) configured, while the cartridge (20) is not inserted in the internal space (30e), to close an opening of an opening part (30a) being a boundary part between the internal space (30e) and an outside of the incubator (30).

7. The culturing system (1, 1a, 1b) according to claim 3, wherein the incubator (30) includes a regulating member (30d) configured, while the cartridge (20) is inserted in the internal space (30e), to regulate a movement in an insertion direction of the cartridge (20), so that a thermal insulation unit (20j) is positioned more outward than the seal structure (30c), the thermal insulation unit (20j) being included in the cartridge (20), being provided between the culturing container (20f) and the culture medium keeping unit (20b), and being configured isolate the culturing container (20f) from the culture medium keeping unit (20b).

8. The culturing system (1, 1a, 1b) according to any one of claims 1 to 7, wherein the cartridge (20) includes a gas supply port (20a1) used for bringing the gas in the internal space (30e) into the culturing container (20f).

9. The culturing system (1, 1a, 1b) according to any one of claims 1 to 8, wherein the cartridge (20) includes a gas discharge port (20a2) used for discharging the gas from an inside of the culturing container (20f).

10. The culturing system (1, 1a, 1b) according to any one of claims 1 to 9, wherein the flow path (20d) is provided with a storage unit (20k) configured to warm the culture medium kept cold in the culture medium keeping unit (20b).

11. The culturing system (1, 1a, 1b) according to any one of claims 1 to 10, wherein the cartridge (20) includes a cold storage unit (20c) configured to keep cold the culture medium kept in the culture medium keeping unit (20b).

12. The culturing system (1, 1a, 1b) according to any one of claims 1 to 10, further comprising a cold storage apparatus (20n), wherein
the cold storage apparatus (20n) is configured, in a state where the cartridge (20) is inserted in the cold storage apparatus (20n), to keep cold the culture medium kept in the culture medium keeping unit (20b).

13. The culturing system (1, 1a, 1b) according to any one of claims 1 to 12, further comprising: an observation apparatus (40) used at a time of observing an inside of the culturing container (20f), through an observation window (30f) included in the incubator (30).

14. The culturing system (1a, 1b) according to claim 3, wherein
the incubator (30) includes:
a first thermometer (32a) configured to measure a first temperature of the internal space (30e);
a hygrometer (32b) configured to measure the humidity of the internal space (30e);
a second thermometer (32c) configured to measure a second temperature of the seal structure (30c);
a heater (32d) configured to heat the seal structure (30c);
a calculating unit (32e) configured to calculate a dew point of the internal space (30e) on a basis of the first temperature measured by the first thermometer (32a) and the humidity measured by the hygrometer (32b); and
a controlling unit (32f) configured to control the heater (32d) so that the second temperature measured by the second thermometer (32c) becomes equal to or higher than the dew point.

15. The culturing system (1a, 1b) according to claim 14, wherein the controlling unit (32f) is configured to control the heater (32d) so that the second temperature measured by the second thermometer (32c) becomes equal to or higher than the dew point and equal to or lower than the first temperature measured by the first thermometer (32a).

16. The culturing system (1a, 1b) according to claim 14 or 15, wherein
the first thermometer (32a) is provided in a position within the internal space (30e) while being positioned apart from the seal structure (30c) by a distance in a range from 35 millimeters or more to 100 millimeters or less inclusive, and
the hygrometer (32b) is provided in a position within the internal space (30e) while being positioned apart from the seal structure (30c) by a distance in a range from 35 millimeters or more to 100 millimeters or less inclusive.

17. The culturing system (1a, 1b) according to claim 14 or 15, wherein
the incubator (30) includes:
a gas introduction port (32h) through which the gas of which the one or both of the temperature and the humidity has been adjusted is introduced; and
a fan (32g) including a suction port through which the gas is sucked from an inside of the internal space (30e) and a blow-out port through which the sucked gas is blown out, wherein
the first thermometer (32a) is provided in a position within the internal space (30e), while being positioned apart from the gas introduction port (32h) by a distance in a range from 30 millimeters or more to 80 millimeters or less inclusive or being positioned apart from the blow-out port by a distance in a range from 30 millimeters or more to 80 millimeters or less inclusive, and
the hygrometer (32b) is provided in a position within the internal space (30e), while being positioned apart from the gas introduction port (32h) by a distance in a range from 30 millimeters or more to 80 millimeters or less inclusive or being positioned apart from the blow-out port by a distance in a range from 30 millimeters or more to 80 millimeters or less inclusive.

18. The culturing system (1a, 1b) according to any one of claims 14 to 16, wherein the first thermometer (32a) is provided in a position closer to the seal structure (30c) than the hygrometer (32b) is.

19. The culturing system (1a, 1b) according to claim 14 or 15, wherein, in a state where the cartridge (20) is inserted in the internal space (30e), the second thermometer (32c) is positioned apart from an abutment part where the cartridge (20) abuts against the seal structure (30c), by a distance in a range from 10 millimeters or more to 20 millimeters or less inclusive.

20. The culturing system according to claim 3, wherein
the seal structure (30c) includes a plurality of seal structures (30c1 to 30c4) respectively corresponding to a plurality of spaces included in the internal space (30e), and
the incubator (30) includes:
a plurality of first thermometers (32a1 to 32a4) each of which is configured to measure the first temperature of a corresponding one of the plurality of spaces;
a plurality of hygrometers (32b1 to 32b4) each of which is configured to measure the humidity of a corresponding one of the plurality of spaces;
a plurality of second thermometers (32c1 to 32c4) each of which is configured to measure the second temperature of a corresponding one of the plurality of seal structures (30c1 to 30c4);
a plurality of heaters (32d1 to 32d4) each of which is configured to heat a corresponding one of the plurality of seal structures (30c1 to 30c4);
a calculating unit (32e) configured to calculate a dew point of each of the plurality of spaces, on a basis of the measured first temperature and the measured humidity; and
a controlling unit (32f) configured to control each of the plurality of heaters (32d1 to 32d4) so that the second temperature measured with respect to each of the plurality of seal structures (30c1 to 30c4) becomes equal to or higher than a corresponding one of the dew points.
